# EUROPEAN PATENT SPECIFICATION

(11) **EP 3 258 266 B1**
(45) Date of publication and mention of the grant of the patent: **15.07.2020**
(21) Application number: 15881482.2
(22) Date of filing: 10.02.2015
(51) Int. Cl.: G01N 33/543, G01N 33/58

(54) **REAGENT KIT USED FOR DETECTING GASTRIN-17, AND PREPARATION METHOD AND APPLICATION FOR REAGENT KIT**
REAGENZIENKIT ZUR DETEKTION VON GASTRIN-17 VERWENDETES REAGENZIENKIT UND HERSTELLUNGSVERFAHREN UND ANWENDUNG FÜR DAS REAGENZIENKIT
NÉCESSAIRE DE RÉACTIFS UTILISÉ POUR LA DÉTECTION DE GASTRINE-17, PROCÉDÉ DE PRÉPARATION ET APPLICATION POUR NÉCESSAIRE DE RÉACTIFS

(43) Date of publication of application: 20.12.2017
(73) Proprietor: Shenzhen New Industries Biomedical Engineering Co. Ltd., Shenzhen, Guangdong 518122 (CN)
(72) Inventor: RAO, Wei, Shenzhen Guangdong 518122 (CN); SUN, Pu, Shenzhen Guangdong 518122 (CN); LI, Yunxuan, Shenzhen Guangdong 518122 (CN); LIU, Wang, Shenzhen Guangdong 518122 (CN); YANG, Yali, Shenzhen Guangdong 518122 (CN); YUAN, Jinyun, Shenzhen Guangdong 518122 (CN); LI, Tinghua, Shenzhen Guangdong 518122 (CN)
(74) Representative: Petraz, Gilberto Luigi
(86) International application number: PCT/CN2015/072682
(87) International publication number: WO 2016/127320

(56) References cited:
- CN-A- 1 781 023
- CN-A- 101 063 684
- CN-A- 101 661 036
- CN-A- 102 426 236
- CN-A- 102 662 049
- CN-A- 104 089 949
- US-B2- 6 696 262
- QIAN-YUN ZHANG ET AL: "Comparison of chemiluminescence enzyme immunoassay based on magnetic microparticles with traditional colorimetric ELISA for the detection of serum [alpha]-fetoprotein", JOURNAL OF PHARMACEUTICAL ANALYSIS,, vol. 2, no. 2, 1 April 2012 (2012-04-01), pages 130-135, XP002772916, ISSN: 2095-1779, DOI: 10.1016/J.JPHA.2011.10.001 [retrieved on 2012-01-26]
- Pia Rinkinen: "GASTRIN-17", , 3 December 2013 (2013-12-03), XP055475254, Retrieved from the Internet: URL:http://www.biohithealthcare.com/resour ce/files/media/manuals/g17-ifu.pdf [retrieved on 2018-05-15]

## Description

### TECHNICAL FIELD

The present disclosure relates to the technical field of biological substance detection, and more particularly, to a kit for detecting gastrin-17 and a preparation method thereof, and a method for detecting gastrin-17 using the kit. The scope of protection is defined by the appended claims, while all embodiments mentioned in this specification encompass that the trace marker directly labels the first or second anti-gastrin-17 antibody, and wherein the first or second anti-gastrin-17 antibody is directly coated on the magnetic sphere.

### BACKGROUND

Gastrin is an important gastrointestinal hormone, secreted by G cells (gastrin containing cell) present on gastric pyloric mucosa into the blood and acting on the stomach to promote gastric secretion. G cells are mainly distributed in the gastric antrum, metabolized in the kidney, and increased in serum during renal dysfunction. Gastrin is secreted when gastric pyloric antrum receives mechanical stimulus such as food and the pyloric pH becomes alkaline. As a result, the secretion of gastric juice, particularly hydrochloric acid, became vigorous. When the pH in the stomach is lowered, the secretion of hydrochloric acid and gastrin is stopped. In addition, gastrin also functions to promote the secretion of pepsin, pancreatic juice, bile, insulin, and the like, though such effects are not strong.

As with translation and maturation of progastrin in the cells, the G cells in the antrum begin to release in the circulation mixed substances of various kinds of gastrin and other precursors produced under acid stimulation. This mixture consists of gastrin-71, -52, -34, -17, -14, and -6, the main forms of which present in serum/plasma are gastrin-34 and gastrin-17. Gastrin-17 (G-17) is the predominant effective form of gastrin in healthy antrum mucosa and is produced almost entirely by G cells. Gastrin-17 is a polypeptide consisting of 17 amino acid residues of two forms: tyrosine residues substituted by sulfate and tyrosine residues unsubstituted by sulfate (gastrin I and II), though these two forms have no difference in physiological activity. The activity of gastrin was found only in the tetrapeptide (Tyr-Met-Asp-Phe-NH2) corresponding to the C-terminal portion, called tetrapeptide gastrin. Studies of the primary structure of gastrin in various mammals have shown that all gastrin-17 are composed of 17 amino acids and varies by animal only in amino acids at positions 5 and 10 from the N terminal. In addition, one of the most important characteristics of gastrin-17 is that all tyrosines at position 12 binds to -SO₃H.

Patients suffering from secondary atrophic gastritis of helicobacter pylori infection generally have a very low level of gastrin-17, corresponding to a risk of gastric cancer and peptic ulcer disease increased to 18 times and 25 times of an average person. Gastrin-17 plays a fundamental or important role in the pathogenesis of gastroduodenal ulcer. For these patients, in the basic state, the serum gastrin is relatively high with an average level of about 160pg/ml and potential overlap with the normal range, but may be significantly increased during feeding and other situations that stimulate secretion of gastrin. High gastrin is one of the important pathogenesis of gastric ulcer. In addition, unusually high levels of gastrin-17 can be regarded as signs of gastric acid deficiency. Gastrin-17 tests can also be used as a postoperative monitoring indicator for a patient, whereas the gastrin-17 secretion level is basically close to 0 after a successful gastric antral resection. In the blood of patients with gastrinoma, i.e., Zollinger-Ellison syndrome, the gastrin-17 concentration is relatively high. Gastrinoma is the most common endocrine tumor of the pancreas, which is a result of proliferation of D cells that secretes gastrin in the pancreas islet. The D cells secret a large amount of gastrin so that the parietal cells increased dramatically. The next places for gastrinoma to occur are the stomach and duodenum. The Zollinger-Ellison syndrome shows the following trilogy: hypergastrinemia, up to 1000pg/ml; high excretion of gastric acid, with basal gastric acid> 15mmol/h, up to 6 times of the normal; and, repeated attacks of ulcer in the stomach and duodenal, mostly refractory ulcers with chronic diarrhea.

At present, detection of gastrin-17 is widely used in clinic, and the main detection method on the market is enzyme-linked immunosorbent assay (ELISA). CN 101063684A discloses a kit comprising 2 different anti-gastrin-17 antibodies, whereas one is coupled to a nanoparticle and the second is detectably labeled.

Enzyme-linked immunosorbent assay (ELISA) is a qualitative and quantitative method, which binds a soluble antigen or antibody to a solid-phase carrier such as polystyrene for immunoreaction due to antigen-antibody binding specificity. The main detection mechanism is the immune sandwich method: one of the gastrin-17-specific capture antibodies is adsorbed on a coated plate, while another test antibody is labeled with horseradish peroxidase (HRP). The HRP-linked monoclonal antibody and gastrin-17 are bound to the solid-phase carrier, incubated, washed, added with a substrate which undergoes color reaction catalyzed by HRP, and ultimately determined for the absorbance by a microplate reader to calculate the concentration of gastrin-17 in the sample.

Enzyme-linked immunosorbent assay requires a long detection time of at least 6 hours or even overnight reaction to complete the test. At the same time, it generally relies on pure manual operation for sample loading and the like, which is low in efficiency and prone to increased deviation in experimental results. Enzymatic reaction may not be sufficiently thorough while is susceptible to external disturbances such as temperature, time, and material concentrations, rendering low specificity and poor sensitivity for detection. In addition, the solid-phase carrier of the enzyme-linked immunosorbent assay is solid and uniformly coated on the wells of the ELISA plate. Pre-dilution of subject sample prior to test is required, which increases the difficulty of the experiment and further reduces reagent sensitivity and accuracy.

In addition, there are methods for detecting full-fragment gastrin currently on the market, mainly radioimmunoassay (RIA) and chemiluminescence immunoassay (CLIA). Although the detection of full-fragment gastrin can prevent missed detection during clinical detection of gastrin content, the gastrin is too complex in composition for every component to be used in clinical disease testing and diagnosis, while polyclonal antibodies used in the detection of full-fragment gastrin may seriously reduce the specificity and sensitivity of reagents, resulting in inaccurate test results.

### SUMMARY

It is an object of the present disclosure to provide a kit for detecting gastrin-17 and a method for preparation the same. Using the specific binding of gastrin-17 to an anti-gastrin-17 antibody in combination with the luminescent properties of trace marker and magnetism of magnetic spheres, the kit can accurately determine the gastrin-17 content of a sample.

It is also an object of the present disclosure to provide a method for detecting gastrin-17, which utilizes the kit provided herein to accurately determine the gastrin-17 content in a sample according to the mechanism of double antibody sandwich or the mechanism of competition. In particular, the use of the kit in automatic chemiluminescence detection of gastrin-17 can shorten the operating time and reduce manual operational errors.

According to the present disclosure, there is provided a kit for detecting gastrin-17 comprising a component A and a component B, wherein the component A is a first anti-gastrin-17 antibody labeled with a trace marker or coated on a magnetic sphere, the component B is a second anti-gastrin-17 antibody or gastrin-17 labeled with a trace marker or coated on a magnetic sphere; wherein either one of the components A and B is labeled with a trace marker and the other one is coated on a magnetic sphere; and, wherein the first anti-gastrin-17 antibody and the second anti-gastrin-17 antibody have different binding sites for binding with gastrin-17.

In the present disclosure, the difference between the first anti-gastrin-17 antibody and the second anti-gastrin-17 antibody lies in their binding sites on gastrin-17. In the kit of the present disclosure, for example, if the first anti-gastrin-17 antibody is coated on a magnetic sphere for binding to gastrin-17 in a subject sample, then the second anti-gastrin-17 antibody labeled with a trace marker is used to bind with another region on gastrin-17 that is different from the binding region of the first anti-gastrin-17 antibody on gastrin-17.

It should be appreciated that in the kit provided herein, the first anti-gastrin-17 antibody may be one or more anti-gastrin-17 antibodies and the second anti-gastrin-17 antibody may be one or more anti-gastrin-17 antibodies, as long as the one or more anti-gastrin-17 antibodies constituting the first anti-gastrin-17 antibodies and the one or more anti-gastrin-17 antibodies constituting the second anti-gastrin-17 antibodies are capable of binding to different binding regions on gastrin-17, respectively.

According to the present disclosure, the first anti-gastrin-17 antibody and the second anti-gastrin-17 antibody may be anti-gastrin-17 monoclonal antibody and/or anti-gastrin-17 polyclonal antibody.

In the human body, more than 95% of the biologically active gastrin is d-amidated gastrin, of which 80% -90% is gastrin-17. Simple detection of gastrin-17 with use of highly-specific monoclonal antibodies can provide better specificity and sensitivity with a simple detection method. Accordingly, the present disclosure provides a kit for measuring the content of gastrin-17 *in vivo,* and the gastrin-17 content can be measured by a kit to reflect occurrence of clinically different conditions.

According to the present disclosure, the trace marker may be selected from trace markers commonly used in the art for labeling antigens or antibodies, such as adamantane, luminol and its derivatives, isoluminol and its derivatives, acridinium esters, alkaline phosphatase (ALP) or horseradish peroxidase (HRP), and N-(4-aminobutyl) -N-ethylisoluene (ABEI) is particularly preferable.

Magnetic spheres suitable for use in the present disclosure are also known as magnetic beads and may be magnetic microspheres commonly used in the art. It is preferable that the magnetic spheres used in the present disclosure are microscale solid-phase microspheres with superparamagnetism and extremely large capacity of protein adsorption formed by combining nanoscale Fe₂O₃ or Fe₃O₄ magnetic particles and an organic polymeric material. Such magnetic spheres can be quickly magnetized in an applied magnetic field while having a remanence of zero after removal of the magnetic field. There is no particular limitation on the types of the organic polymeric material, which may be selected as necessary.

The magnetic microspheres used in the present disclosure should have a diameter of 0.1 to 5 microns, and the magnetic microspheres may be surface modified to carry a variety of active functional groups including, but not limited to, -OH, -COOH, -NH₂.

In a specific embodiment, the magnetic sphere is a complex of Fe₂O₃ or Fe₃O₄ magnetic particles and an organic polymeric material and has a particle size of 0.1 to 5 microns; and, the magnetic spheres are optionally modified by surface modification to carry one or more active functional groups.

According to the present disclosure, the concentrations of the first anti-gastrin-17 antibody is 10 to 200 µg/ml, and the second anti-gastrin-17 antibody or gastrin-17 in the kit are 10 to 200 µg/ml, respectively, the concentration of the trace marker is 0.1 to 1 mg/ml, and the concentration of the magnetic spheres is 0.1 to 5 mg/ml. The concentration for each component above is based on the amount of each independent component containing such component.

According to the present disclosure, the kit further comprises a low-point calibrator and a high-point calibrator of gastrin-17 and optionally a buffer. The low-point calibrator and high-point calibrator herein is referred to relative to each other, wherein the "low-point calibrator" refers to a calibrator made by diluting gastrin-17 to a concentration of 10 to 30 ng/ml using a 50% bovine serum product, and the "high-point calibrator" refers to a calibrator made by diluting gastrin-17 to a concentration of 500 to 700 ng/ml using a 50% bovine serum product. The low-point calibrator and high-point calibrator each optionally contains bovine serum albumin (BSA) and/or a preservative. The BSA concentration is preferably 0.01 to 0.5 g/ml.

In some embodiments of the present disclosure, the component B is the second anti-gastrin-17 antibody coated on a magnetic sphere or labeled with a trace marker.

In these embodiments, the trace marker directly or indirectly labels the first anti-gastrin-17 antibody or the second anti-gastrin-17 antibody. Indirect labeling comprises, but is not limited to, indirect labeling via a fluorescein isothiocyanate (FITC) and anti-FITC antibody system or a streptavidin (SA) and biotin system. Direct labeling means that ABEI is directly linked to the first anti-gastrin-17 antibody or the second anti-gastrin-17 antibody; indirect labeling means that ABEI is indirectly linked to the first anti-gastrin-17 antibody or the second anti-gastrin-17 antibody via an intermediate medium linking system, comprising, but not limited to, a FITC and anti-FITC antibody system or a streptavidin and biotin system. The anti-FITC antibody is preferably a goat anti-FITC polyclonal antibody.

In these embodiments, the first anti-gastrin-17 antibody or the second anti-gastrin-17 antibody is directly or indirectly coated on magnetic spheres. Indirect coating forms of the magnetic sphere include, but are not limited to, indirect coating by a FITC and anti-FITC antibody system or a streptavidin and biotin system. Direct coating refers to coating the magnetic spheres directly using the first anti-gastrin-17 antibody or the second anti-gastrin-17 antibody; and indirect coating refers to coating the magnetic spheres using the first anti-gastrin-17 antibody or the second anti-gastrin-17 antibody via an intermediate medium linking system including, but not limited to, a FITC and anti-FITC antibody system or a streptavidin and biotin system.

In a specific embodiment, the gastrin-17 detection kit comprises the following components: 1) a solution of a first anti-gastrin-17 antibody labeled with a trace marker, wherein the concentration of the first anti-gastrin-17 antibody is 10 to 200 µg/ml and the concentration of the trace marker is 0.1 to 1 mg/ml; 2) a suspension of magnetic spheres coated with a second anti-gastrin-17 antibody, wherein the concentration of the second anti-gastrin-17 antibody is 10 to 200 µg/ml and the concentration of the magnetic sphere is 0.1 to 5 mg/ml; (3) a low-point calibrator containing gastrin-17 with a gastrin-17 concentration of 2 to 50 ng/ml; and 4) a high-point calibrator containing gastrin-17 with a gastrin-17 concentration of 200 to 1000ng/ml.

In a specific embodiment, the gastrin-17 detection kit comprises the following components: 1) a solution of a first anti-gastrin-17 antibody labeled with a trace marker, wherein the concentration of the first anti-gastrin-17 antibody is 10 to 200 µg/ml and the concentration of the trace marker is 0.1 to 1 mg/ml; 2) a solution of a second anti-gastrin-17 antibody labeled with FITC, wherein the concentration of the second anti-gastrin-17 antibody is 10 to 200 µg/ml and the concentration of FITC is 0.1 to 1 mg/ml; (3) a suspension of magnetic spheres coated with a goat anti-FITC antibody, wherein the concentration of the goat anti-FITC antibody is 0.1 to 1 mg/ml and the concentration of the magnetic sphere is 0.1 to 5 mg/ml; (4) a low-point calibrator containing gastrin-17 with a gastrin-17 concentration of 2 to 50 ng/ml; and (5) a high-point calibrator containing gastrin-17 with a gastrin-17 concentration of 200 to 1000ng/ml.

In a specific embodiment, the gastrin-17 detection kit comprises the following components: (1) a suspension of magnetic spheres coated with streptavitin, the concentration of streptavitin is 10 to 200 µg/ml and the concentration of the magnetic sphere is 0.1 to 5 mg/ml; (2) a solution of a first anti-gastrin-17 antibody labeled with a trace marker, wherein the concentration of the first anti-gastrin-17 antibody is 10 to 200 µg/ml and the concentration of the trace marker is 0.1 to 1 mg/ml; (3) a biotinylated solution of a second anti-gastrin-17 antibody, wherein the concentration of the second anti-gastrin-17 antibody is 10 to 200 µg/ml and the concentration of biotin is 0.1 to 1 mg/ml; (4) a low-point calibrator containing gastrin-17 with a gastrin-17 concentration of 2 to 50 ng/ml; and (5) a high-point calibrator containing gastrin-17 with a gastrin-17 concentration of 200 to 1000ng/ml.

In a specific embodiment, the gastrin-17 detection kit comprises the following components: (1) a suspension of magnetic spheres coated with a second anti-gastrin-17 antibody, the concentration of the second anti-gastrin-17 antibody is 10 to 200 µg/ml and the concentration of the magnetic sphere is 0.1 to 5 mg/ml; (2) a biotinylated solution of a first anti-gastrin-17 antibody, wherein the concentration of the first anti-gastrin-17 antibody is 10 to 200 µg/ml and the concentration of biotin is 0.1 to 1 mg/ml;; (3) a solution of streptavitin labeled with a trace marker, wherein the concentration of streptavitin is 10 to 200 µg/ml and the concentration of the trace marker is 0.1 to 1 mg/ml; (4) a low-point calibrator containing gastrin-17 with a gastrin-17 concentration of 2 to 50 ng/ml; and (5) a high-point calibrator containing gastrin-17 with a gastrin-17 concentration of 200 to 1000ng/ml.

In each of the specific embodiments above, the components of the kit preferably each contain BSA and a preservative; the BSA concentration is preferably 0.01 to 0.5 g/ml; the preservative is any one or a mixture of any two or more selected from the group consisting of potassium sorbate, sodium benzoate, sodium azide, sodium nitrite, and the Proclin Series (one of the commonly used preservatives for immunodiagnosis, the main active ingredients being 2-methyl-4-isothiazolin-3-one and 5-chloro-2-methyl-4-isothiazolin-3-one). The gastrin-17 antibody may be monoclonal or polyclonal, and the trace marker may be any one selected from the group consisting of acridinium esters, isoluminol and its derivatives, luminol and its derivatives, ALP, or HRP.

In some other embodiments, the component B is gastrin-17 coated on a magnetic sphere or labeled with a trace marker.

In these embodiments, the trace marker directly or indirectly labels the first anti-gastrin-17 antibody or gastrin-17. Indirect labeling includes, but is not limited to, indirect labeling with a fluorescein isothiocyanate (FITC) and anti-FITC antibody system or a streptavidin and biotin system. Direct labeling means that ABEI is directly linked to the first anti-gastrin-17 antibody or gastrin-17, while indirect labeling means that ABEI labels the first anti-gastrin-17 antibody or gastrin 17 via an intermediate medium linking system, which includes, but is not limited to, a FITC and anti-FITC antibody system or a streptavidin and biotin system.

In these embodiments, the first anti-gastrin-17 antibody or gastrin-17 is directly or indirectly coated on a magnetic sphere. The forms of indirect coating the magnetic spheres comprises, but is not limited to, indirect coating via a FITC and anti-FITC antibody system or a streptavidin and biotin system. Direct coating refers to the direct coating of magnetic spheres using the first anti-gastrin-17 antibody or gastrin-17, while indirect coating refers to the use of an intermediate medium linking system to coat the first anti-gastrin-17 antibody or gastrin-17 on the magnetic spheres, the intermediate medium linking system including, but is not limited to, a FITC and anti-FITC antibody system or a streptavidin and biotin system.

In some embodiments, the component A is the (one or more) first anti-gastrin-17 antibody (s) labeled with a trace marker, and the component B is gastrin-17 coated on magnetic spheres.

In a specific embodiment, the gastrin-17 detection kit comprises the following components: 1) a solution of an anti-gastrin-17 antibody labeled with a trace marker, wherein the concentration of the first anti-gastrin-17 antibody is 10 to 200 µg/ml and the concentration of the trace marker is 0.1 to 1 mg/ml; (2) a suspension of magnetic spheres coated with gastrin-17 antigen, wherein the concentration of the gastrin-17 antigen is 10 to 200 µg/ml and the concentration of the magnetic sphere is 0.1 to 5 mg/ml; (3) a low-point calibrator containing gastrin-17 with a gastrin-17 concentration of 2 to 50 ng/ml; and 4) a high-point calibrator containing gastrin-17 with a gastrin-17 concentration of 200 to 1000ng/ml.

In a specific embodiment, the gastrin-17 detection kit comprises the following components: 1) a solution of an anti-gastrin-17 antibody labeled with a trace marker, wherein the concentration of the anti-gastrin-17 antibody is 10 to 200 µg/ml and the concentration of the trace marker is 0.1 to 1 mg/ml; (2) a solution of gastrin-17 antigen labeled with FITC, wherein the concentration of the gastrin-17 antigen is 10 to 200 µg/ml and the concentration of FITC is 0.1 to 1 mg/ml; (3) a suspension of magnetic spheres coated with a goat anti-FITC antibody, wherein the concentration of the goat anti-FITC antibody is 0.1 to 1 mg/ml and the concentration of the magnetic sphere is 0.1 to 5 mg/ml; (4) a low-point calibrator containing gastrin-17 with a gastrin-17 concentration of 2 to 50 ng/ml; and (5) a high-point calibrator containing gastrin-17 with a gastrin-17 concentration of 200 to 1000ng/ml.

In a specific embodiment, the gastrin-17 detection kit comprises the following components: (1) a solution of an anti-gastrin-17 antibody labeled with a trace marker, wherein the concentration of the anti-gastrin-17 antibody is 10 to 200 µg/ml and the concentration of the trace marker is 0.1 to 1 mg/ml; (2) a solution of gastrin-17 antigen labeled with biotin, wherein the concentration of the gastrin-17 antigen is 10 to 200 µg/ml and the concentration of biotin is 0.1 to 1 mg/ml;; (3) a suspension of magnetic spheres coated with streptavitin, the concentration of streptavitin is 10 to 200 µg/ml and the concentration of the magnetic sphere is 0.1 to 5 mg/ml; (4) a low-point calibrator containing gastrin-17 with a gastrin-17 concentration of 2 to 50 ng/ml; and (5) a high-point calibrator containing gastrin-17 with a gastrin-17 concentration of 200 to 1000ng/ml.

In a specific embodiment, the gastrin-17 detection kit comprises the following components: (1) a suspension of magnetic spheres coated with an anti-gastrin-17 antibody, the concentration of the second anti-gastrin-17 antibody is 10 to 200 µg/ml and the concentration of the magnetic sphere is 0.1 to 5 mg/ml; (2) a solution of a gastrin-17 antigen labeled with biotin, wherein the concentration of the first anti-gastrin-17 antibody is 10 to 200 µg/ml and the concentration of biotin is 0.1 to 1 mg/ml;; (3) a solution of streptavitin labeled with a trace marker, wherein the concentration of streptavitin is 10 to 200 µg/ml and the concentration of the trace marker is 0.1 to 1 mg/ml; (4) a low-point calibrator containing gastrin-17 with a gastrin-17 concentration of 2 to 50 ng/ml; and (5) a high-point calibrator containing gastrin-17 with a gastrin-17 concentration of 200 to 1000ng/ml.

In each of the specific embodiments above, the components of the kit preferably each contain BSA and a preservative; the BSA concentration is preferably 0.01 to 0.5 g/ml; the preservative is any one or a mixture of any two or more selected from the group consisting of potassium sorbate, sodium benzoate, sodium azide, sodium nitrite, and the Proclin Series. The gastrin-17 antibody may be monoclonal or polyclonal, and the trace marker may be any one selected from the group consisting of acridinium esters, isoluminol and its derivatives, luminol and its derivatives, ALP, or HRP.

The present disclosure provides a method for preparing a kit with the component B as a second anti-gastrin-17 antibody as described above, the method comprising: directly or indirectly labeling either one of a first anti-gastrin-17 antibody and a second anti-gastrin-17 antibody with a trace marker and directly or indirectly coating the other one on a magnetic sphere. The indirect labeling comprises, but is not limited to, labeling the first or second anti-gastrin-17 antibody with the trace marker via a fluorescein isothiocyanate and anti-fluorescein isothiocyanate antibody system or a streptavidin and biotin system; and the indirect coating comprises, but is not limited to, coating the magnetic sphere with the first or second anti-gastrin-17 antibody via a fluorescein isothiocyanate and anti-fluorescein isothiocyanate antibody system or a streptavidin and biotin system.

The present disclosure provides a method for preparing a kit with the component B as a gastrin-17 as described above, the method comprising: directly or indirectly labeling either one of a first anti-gastrin-17 antibody and gastrin-17 with a trace marker and directly or indirectly coating the other one on a magnetic sphere. The indirect labeling comprises, labeling the first anti-gastrin-17 antibody or gastrin-17 with the trace marker via a fluorescein isothiocyanate and anti-fluorescein isothiocyanate antibody system or a streptavidin and biotin system; and the indirect coating comprises, but is not limited to, coating the magnetic sphere with the first anti-gastrin-17 antibody or gastrin-17 via a fluorescein isothiocyanate and anti-fluorescein isothiocyanate antibody system or a streptavidin and biotin system.

The method for preparing a kit according to the present disclosure may further comprise preparing a low-point calibrator and a high-point calibrator, and may further comprise assembling the kit.

According to the present disclosure, there is also provided a method for detecting gastrin-17, which comprises detecting gastrin-17 concentration in a subject sample by chemiluminescence immunoassay using a kit as described above.

In particular, the method may comprise: mixing the components A and B of the kit with the subject sample, incubating, magnetically separating, and adding a luminescent substrate to a resulting precipitate to detect an optical signal intensity; measuring an optical signal intensity of the low-point calibrator and the high-point calibrator of the gastrin-17 in the same manner to obtain a standard curve between the gastrin-17 concentration and the optical signal intensity; and, comparing the optical signal intensity of the subject sample with the standard curve to obtain the gastrin-17 concentration in the subject sample.

In the case where the component B of the kit is a second anti-gastrin-17 antibody, when determining gastrin-17 using the kit, the components A and B form a double antibody sandwich mode of the first anti-gastrin-17 antibody/gastrin-17/the second anti-gastrin-17 antibody with gastrin-17 in the subject sample, that is, forming an immune complex of an anti-gastrin-17 antibody labeled with a trace marker, gastrin-17 to be detected, and gastrin-17 antibody coated on the magnetic spheres. External magnetic field is applied for precipitation, after which the supernatant is removed, and the precipitate complex is washed with washing buffer and added with luminous excitant for determination of relative luminous intensity. The gastrin-17 concentration in the subject sample can be obtained with reference to the standard curve of gastrin-17 concentration and luminous intensity.

In a specific embodiment, determination is performed with a double antibody sandwich assay, and the specific sample loading procedure is: a. adding 15 to 100 µl of a subject sample (serum/plasma sample), the high-point calibrator, and the low-point calibrator to different assay wells; b. adding 10 to 50µl of the suspension of magnetic spheres coated with anti-gastrin-17 antibody; c. adding 40 to 200 µl of the solution of another anti-gastrin-17 antibody labeled with a trace marker; d. incubating under 37°C for 15 to 40 minutes, and cleaning in a magnetic environment for at least 2 times; e. adding luminescent substrate and detecting optical signal intensity; and f. calculating the gastrin-17 concentration in the subject sample using the optical signal intensity detected in the subject sample with reference to the calibrated working curve with the calibrator.

In the case where the component B of the kit is gastrin-17, when determining gastrin-17 using the kit, gastrin-17 in the component B competes with gastrin-17 in the subject sample to bind with the first anti-gastrin-17 antibody in the component A, forming an immune complex. External magnetic field is applied for precipitation, after which the supernatant is removed, and the precipitate complex is washed with washing buffer and added with luminous excitant for determination of relative luminous intensity. The gastrin-17 concentration in the subject sample can be obtained with reference to the standard curve of gastrin-17 concentration and luminous intensity.

In a specific embodiment, determination is performed with a competition assay, and the specific sample loading procedure is: a. adding 15 to 100 µl of a subject sample (serum/plasma sample), the high-point calibrator, and the low-point calibrator to different assay wells; b. adding 10 to 50µl of the suspension of magnetic spheres coated with gastrin-17 antigen; c. adding 40 to 200 µl of the solution of anti-gastrin-17 antibody labeled with a chemical trace marker; d. incubating under 37°C for 15 to 40 minutes, and cleaning in a magnetic environment for at least 2 times; e. adding luminescent substrate and detecting optical signal intensity; and f. calculating the gastrin-17 concentration in the subject sample using the optical signal intensity detected in the subject sample with reference to the calibrated working curve with the calibrator.

In one embodiment, the method for detecting gastrin-17 concentration comprises detecting gastrin-17 concentration by a chemiluminescence immunoassay analyser using a kit as described above. In a preferred embodiment of the present disclosure, the method is performed in full automation. According to the present disclosure, the chemiluminescence immunoassay analyser is preferably a Maglumi series chemiluminescence immunoassay analyzer (manufactured by Shenzhen New Industries Biomedical Engineering Co., Ltd.). When using a chemiluminescence immunoassay analyser, the analyser can automatically add samples, detect, and calculate results according to the settings, speeding up the detection process and reducing human errors.

The gastrin-17 detection kit provided herein comprises gastrin-17 or anti-gastrin-17 antibody labeled with a trace marker in various labeling forms or coated on magnetic microspheres in various coating forms and is capable of accurate, sensitive, and rapid determination of the gastrin-17 concentration in a sample by double antibody sandwich method or competition method.

The use of the kit provided herein for determination of gastrin-17 does not require dilution of subject sample which can be used directly for detection, thereby facilitating detection sensitivity of a low-level sample. The subject sample may be any one of a blank tube serum, separation gel tube serum, coagulant tube serum, EDTA plasma, and heparin plasma.

The method for detecting gastrin-17 provided herein has greatly improved the specificity and sensitivity of a kit by using a more advanced method of chemiluminescence immunoassay for better clinical diagnosis.

The method for detecting gastrin-17 provided herein uses the kit according to the present disclosure and chemiluminescence immunoassay and performs sample loading with full automation of the instrument, which reduces the interference caused by human factors to the experimental results and greatly shortens the test time, facilitating rapid results for clinical diagnosis.

### DETAILED DESCRIPTION OF THE INVENTION

The present disclosure will now be further described by way of specific embodiments and specific examples, and it is to be understood that the scope of the disclosure is not limited thereto.

The following examples employed a Maglumi 2000 plus chemiluminescence immunoassay analyzer (manufactured by Shenzhen New Industries Biomedical Engineering Co., Ltd.) for detection.

Source of gastrin-17 enzyme-linked immunodetection kit: Biohit (Finland), Catalog No.: 18GC1404.

Source of anti-gastrin-17 antibody: both antibodies (the first anti-gastrin-17 antibody and the second anti-gastrin-17 antibody) were purchased from Biohit (Finland), including the first anti-gastrin-17 antibody of clone number G52C7.1 and the second anti-gastrin-17 antibody of clone number G55D4.

Source of goat anti-FITC polyclonal antibody: purchased from Beijing Biohao Biological Technology Co., Ltd.

Source of FITC: purchased from Shanghai Jining Shiye Co., Ltd.

Source of ABEI: manufactured by Shenzhen New Industries Biomedical Engineering Co., Ltd.

Source of magnetic microsphere: manufactured by Shenzhen New Industries Biomedical Co., Ltd., with a concentration of 0.6 to 1.2 mg/ml, the 80% particle size distribution of 1 to 5µm, precipitation time of 10 to 15 seconds at a magnetic intensity of 4000 gauss, and protein adsorption concentration of 0.8mg to1.2mg at 30mg BSA.

Source of Biotin and streptavidin: purchased from Shanghai Yuanye Biotechnology Co., Ltd.

Gastrin-17 standards: purchased from Shanghai Science Peptide Biological Technology Co., Ltd.

The methods for preparing the components of the kit are as follows:
**Preparation Example 1: Preparation of a suspension of magnetic spheres coated with the first gastrin-17 antibody**
   The immunomagnetic spheres used in this preparation procedure was a suspension of nano-magnetic microspheres at a concentration of 100 mg/ml with hydroxyl group of 95 mg KOH/g, manufactured by Merck Co., Ltd..
   (1) Preparation of buffer:
      2.55g sodium acetate trihydrate was weighed, dissolved in 4500ml of purified water, added with 14ml acetic acid, and well mixed to produce an acetic acid buffer of pH 3.6.
   (2) Linking of the magnetic microspheres (CMC method for linking magnetic microspheres):
      The magnetic microspheres were suspended in the acetic acid buffer (pH 3.6) above of 5x coating volume to give a magnetic sphere concentration of 20 mg/ml, and 1-cyclohexyl-2-morpholinoethyl-carbodiimide metho-p-toluenesulfonate (CMC) was added to a concentration of 10 mg/ml. Purified first anti-gastrin-17 antibodies were added by a weight ratio of the resultant solution to the first anti-gastrin-17 antibodies at 1mg: 12µg, and underwent reaction in a constant-temperature shaking bath incubator at 37 °C for 24 hours.
   (3) Cleaning of magnetic microspheres:
      Preparation of cleaning solution for magnetic spheres: 500ml PBS buffer (pH 7.4) was formulated with 0.1mol/l PBS buffer and purified water at a volumetric ratio of 1: 9, into which 2.5g BSA was added, well mixed, and dissolved to prepare the cleaning solution for magnetic spheres.
      Cleaning: the magnetic spheres after the warm bath in step (2) were poured into a beaker, placed on magnet for precipitation, had the supernatant removed, washed under stirring with 5x volume of the cleaning solution for magnetic spheres, placed on magnet, and had the cleared supernatant removed. The cleaning procedure was repeated for four times.
   (4) Suspension of the magnetic spheres:
      The magnetic spheres cleaned in step (3) were added to a mixed solution (primary composition of the mixed solution: 0.2 g/ml KH₂PO₄, 2.9 g/ml NaHPO₄, 8g/ml NaCl, 2 g/ml NaN₃, 5 g/ml BSA, 2 ml/ml Twain T-20, balanced with purified water) of 1x coating volume, to obtain a suspension of magnetic spheres of 1x coating volume with a suspension concentration of 20 mg/ml, i.e., the suspension of magnetic spheres coated with the first anti-gastrin-17 antibodies.

   The first anti-gastrin-17 antibodies above can be replaced by the second anti-gastrin-17 antibodies.
**Preparation Example 2: Preparation of a suspension of magnetic spheres coated with streptavidin**
   The immunomagnetic spheres used in this preparation procedure was a suspension of nano-magnetic microspheres at a concentration of 100 mg/ml with hydroxyl group of 95 mg KOH/g, manufactured by Merck Co., Ltd..
   (1) Preparation of buffer:
      2.55g sodium acetate trihydrate was weighed, dissolved in 4500ml of purified water, added with 14ml acetic acid, and well mixed to produce an acetic acid buffer of pH 3.6.
   (2) Linking of the magnetic microspheres (CMC method for linking magnetic microspheres):
      The magnetic microspheres were suspended in the acetic acid buffer (pH 3.6) above of 5x coating volume to give a magnetic sphere concentration of 20 mg/ml, and CMC (1-cyclohexyl-2-morpholinoethyl-carbodiimide metho-p-toluenesulfonate) was added to a concentration of 10 mg/ml. Streptavidin was added by a weight ratio of the resultant solution to streptavidin at 1mg: 12µg, and underwent reaction in a constant-temperature shaking bath incubator at 37 °C for 24 hours.
   (3) Cleaning of magnetic microspheres:
      Preparation of cleaning solution for magnetic spheres: 500ml PBS buffer (pH 7.4) was formulated with 0.1mol/l PBS buffer and purified water at a volumetric ratio of 1: 9, into which 2.5g BSA was added, well mixed, and dissolved to prepare the cleaning solution for magnetic spheres.
      Cleaning: the magnetic spheres after the warm bath in step (2) were poured into a beaker, placed on magnet for precipitation, had the supernatant removed, washed under stirring with 5x volume of the cleaning solution for magnetic spheres, placed on magnet, and had the cleared supernatant removed. The cleaning procedure was repeated for four times.
   (4) Suspension of the magnetic spheres: The magnetic spheres cleaned in step (3) were added to a mixed solution (primary composition of the mixed solution: 0.2 g/ml KH₂PO₄, 2.9 g/ml NaHPO₄, 8g/ml NaCl, 2 g/ml NaN₃, 5 g/ml BSA, 2 ml/ml Twain T-20, balanced with purified water) of 1x coating volume, to obtain a suspension of magnetic spheres of 1x coating volume with a suspension concentration of 20 mg/ml, i.e., the suspension of magnetic spheres coated with streptavitin.
**Preparation Example 3: Preparation of a suspension of magnetic spheres coated with goat anti-FITC polyclonal antibody**
   The immunomagnetic spheres used in this preparation procedure was a suspension of nano-magnetic microspheres at a concentration of 100 mg/ml with hydroxyl group of 95 mg KOH/g, manufactured by Merck Co., Ltd..
   (1) Preparation of buffer:
      2.55g sodium acetate trihydrate was weighed, dissolved in 4500ml of purified water, added with 14ml acetic acid, and well mixed to produce an acetic acid buffer of pH 3.6.
   (2) Linking of the magnetic microspheres (CMC method for linking magnetic microspheres):
      The magnetic microspheres were suspended in the acetic acid buffer (pH 3.6) above of 5x coating volume to give a magnetic sphere concentration of 20 mg/ml, and CMC (1-cyclohexyl-2-morpholinoethyl-carbodiimide metho-p-toluenesulfonate) was added to a concentration of 10 mg/ml. Goat anti-FITC polyclonal antibody were added by a weight ratio of the resultant solution to the goat anti-FITC polyclonal antibody at 1mg: 12µg, and underwent reaction in a constant-temperature shaking bath incubator at 37 °C for 24 hours.
   (3) Cleaning of magnetic microspheres:
      Preparation of cleaning solution for magnetic spheres: 500ml PBS buffer (pH 7.4) was formulated with 0.1mol/l PBS buffer and purified water at a volumetric ratio of 1: 9, into which 2.5g BSA was added, well mixed, and dissolved to prepare the cleaning solution for magnetic spheres.
      Cleaning: the magnetic spheres after the warm bath in step (2) were poured into a beaker, placed on magnet for precipitation, had the supernatant removed, washed under stirring with 5x volume of the cleaning solution for magnetic spheres, placed on magnet, and had the cleared supernatant removed. The cleaning procedure was repeated for four times.
   (4) Suspension of the magnetic spheres:
      The magnetic spheres cleaned in step (3) were added to a mixed solution (primary composition of the mixed solution: 0.2 g/ml KH₂PO₄, 2.9 g/ml NaHPO₄, 8g/ml NaCl, 2 g/ml NaN₃, 5 g/ml BSA, 2 ml/ml Twain T-20, balanced with purified water) of 1x coating volume, to obtain a suspension of magnetic spheres of 1x coating volume with a suspension concentration of 20 mg/ml, i.e., the suspension of magnetic spheres coated with the goat anti-FITC polyclonal antibody.
**Preparation Example 4: Preparation of a solution of the first anti-gastrin-17 antibody labeled with ABEI**
   (1) Preparation of pH 9.5 dialysis solution: in a 5000ml beaker, 14.31g of Na₂CO₃ and 26.46g of NaHCO₃ was added, and filled up to 4500ml with water. The dialysate formulated was placed on a magnetic stirrer for later use.
   (2) A dialysis bag with an interception capacity of 14000 was chosen, a portion of which with appropriate size was prepared for later use. 1mg of anti-gastrin-17 monoclonal or polyclonal antibodies was dissolved and adjusted to 1ml with the dialysis solution formulated above, and placed into the dialysis bag. Dialysis was performed under stirring for 2 hours, and 300 µg of ABEI-hemisuccinimide-N-hydroxysuccinimide was added to the dialyzed solution for reaction at 37 °C for 2 hours to produce the solution of the first anti-gastrin-17 antibodies labeled with ABEI.
   (3) Purification of the solution of the first anti-gastrin-17 antibodies labeled with ABEI obtained in the above reaction was performed on a G-25 gel column.
   (4) An equal volume of 5 g/ml BSA protective solution was added to the purified solution of the first anti-gastrin-17 antibodies labeled with ABEI to obtain the final solution.

   The first anti-gastrin-17 antibodies above can be replaced by the second anti-gastrin-17 antibodies.
**Preparation Example 5: Preparation of a solution of the first anti-gastrin-17 antibody labeled with biotin**
   (1) Preparation of pH 9.5 dialysis solution: in a 5000ml beaker, 14.31g of Na₂CO₃ and 26.46g of NaHCO₃ was added, and filled up to 4500ml with water. The dialysate formulated was placed on a magnetic stirrer for later use.
   (2) A dialysis bag with an interception capacity of 14000 was chosen, a portion of which with appropriate size was prepared for later use. 1mg of the first anti-gastrin-17 antibodies was dissolved and adjusted to 1ml with the dialysis solution formulated above, and placed into the dialysis bag. Dialysis was performed under stirring for 2 hours, and 300 µg of biotin was added to the dialyzed solution for reaction at 37 °C for 2 hours to produce the biotinylated solution of the first anti-gastrin-17 antibodies.
   (3) Purification of the biotinylated solution of the first anti-gastrin-17 antibodies obtained in the above reaction was performed on a G-25 gel column.
   (4) An equal volume of 5 g/ml BSA protective solution was added to the purified biotinylated solution of the first anti-gastrin-17 antibodies to obtain the final solution.

   The first anti-gastrin-17 antibodies above can be replaced by the second anti-gastrin-17 antibodies.
**Preparation Example 6: Preparation of a solution of the first anti-gastrin-17 antibody labeled with FITC**
   (1) Preparation of pH 9.5 dialysis solution: in a 5000ml beaker, 14.31g of Na₂CO₃ and 26.46g of NaHCO₃ was added, and filled up to 4500ml with water. The dialysate formulated was placed on a magnetic stirrer for later use.
   (2) A dialysis bag with an interception capacity of 14000 was chosen, a portion of which with appropriate size was prepared for later use. 1mg of the first anti-gastrin-17 antibody was dissolved and adjusted to 1ml with the dialysis solution formulated above, and placed into the dialysis bag. Dialysis was performed under stirring for 2 hours, and 100 µg of FITC was added to the dialyzed solution for reaction at 37 °C for 2 hours to produce the solution of the first anti-gastrin-17 antibody labeled with FITC.
   (3) Purifying of the solution of FITC-labeled first anti-gastrin-17 antibodies obtained in the above reaction was performed on a G-25 gel column.
   (4) An equal volume of 5 g/ml BSA protective solution was added to the purified solution of FITC-labeled first anti-gastrin-17 antibodies to obtain the final solution.

   The first anti-gastrin-17 antibodies above can be replaced by the second anti-gastrin-17 antibodies.
**Preparation Example 7: Preparation of a solution of the first anti-gastrin-17 antibody labeled with streptavidin**
   (1) Preparation of pH 9.5 dialysis solution: in a 5000ml beaker, 14.31g of Na₂CO₃ and 26.46g of NaHCO₃ was added, and filled up to 4500ml with water. The dialysate formulated was placed on a magnetic stirrer for later use.
   (2) A dialysis bag with an interception capacity of 14000 was chosen, a portion of which with appropriate size was prepared for later use. 1mg of the first anti-gastrin-17 antibodies was dissolved and adjusted to 1ml with the dialysis solution formulated above, and placed into the dialysis bag. Dialysis was performed under stirring for 2 hours, and 50 µg of streptavidin was added to the dialyzed solution for reaction at 37 °C for 2 hours to produce the first anti-gastrin-17 antibodies labeled with streptavidin.
   (3) Purification of the solution of the first anti-gastrin-17 antibodies labeled with streptavidin in the above reaction with a G-25 gel column.
   (4) An equal volume of 5 g/ml BSA protective solution was added to the purified solution of the first anti-gastrin-17 antibodies labeled with streptavidin to obtain the final solution.

   The first anti-gastrin-17 antibodies above can be replaced by the second anti-gastrin-17 antibodies.
**Preparation Example 8: Preparation of a solution of the first anti-gastrin-17 antibody labeled with goat anti-FITC polyclonal antibody**
   (1) Preparation of pH 9.5 dialysis solution: in a 5000ml beaker, 14.31g of Na₂CO₃ and 26.46g of NaHCO₃ was added, and filled up to 4500ml with water. The dialysate formulated was placed on a magnetic stirrer for later use.
   (2) A dialysis bag with an interception capacity of 14000 was chosen, a portion of which with appropriate size was prepared for later use. 1mg of the first anti-gastrin-17 antibodies was dissolved and adjusted to 1ml with the dialysis solution formulated above, and placed into the dialysis bag. Dialysis was performed under stirring for 2 hours, and 50 µg of goat anti-FITC polyclonal antibodies was added to the dialyzed solution for reaction at 37 °C for 2 hours to produce the solution of the first anti-gastrin-17 antibodies labeled with goat anti-FITC polyclonal antibodies.
   (3) Purification of the solution of the first anti-gastrin-17 antibodies labeled with goat anti-FITC polyclonal antibodies obtained in the above reaction was performed on a G-25 gel column.
   (4) An equal volume of 5 g/ml of BSA protective solution was added to the purified solution of the first anti-gastrin-17 antibodies labeled with goat anti-FITC polyclonal antibodies to obtain the final solution.

   The first anti-gastrin-17 antibodies above can be replaced by the second anti-gastrin-17 antibodies.
**Preparation Example 9: Preparation of a biotinylated ABEI solution**
   (1) Preparation of pH 9.5 dialysis solution: in a 5000ml beaker, 14.31g of Na₂CO₃ and 26.46g of NaHCO₃ was added, and filled up to 4500ml with water. The dialysate formulated was placed on a magnetic stirrer for later use.
   (2) A dialysis bag with an interception capacity of 14000 was chosen, a portion of which with appropriate size was prepared for later use. 1mg of biotin was dissolved and adjusted to 1ml with the dialysis solution formulated above, and placed into the dialysis bag. Dialysis was performed under stirring for 2 hours, and 300 µg of ABEI-hemisuccinimide-N-Hydroxysuccinimide was added to the dialyzed solution for reaction at 37 °C for 2 hours to produce the solution of biotinylated ABEI.
   (3) Purification of the biotinylated ABEI solution obtained in the above reaction was performed on a G-25 gel column.
   (4) An equal volume of 5 g/ml BSA protective solution was added to the purified, biotinylated ABEI solution to obtain the final solution.
**Preparation Example 10: Preparation of a solution of ABEI labeled with streptavidin**
   (1) Preparation of pH 9.5 dialysis solution: in a 5000ml beaker, 14.31g of Na₂CO₃ and 26.46g of NaHCO₃ was added, and filled up to 4500ml with water. The dialysate formulated was placed on a magnetic stirrer for later use.
   (2) A dialysis bag with an interception capacity of 14000 was chosen, a portion of which with appropriate size was prepared for later use. 1mg of streptavitin was dissolved and adjusted to 1ml with the dialysis solution formulated above, and placed into the dialysis bag. Dialysis was performed under stirring for 2 hours, and 50 µg of ABEI-hemisuccinimide-N-Hydroxysuccinimide was added to the dialyzed solution for reaction at 37 °C for 2 hours to produce the solution of ABEI labeled with streptavitin.
   (3) Purification of the solution of ABEI labeled with streptavitin obtained in the above reaction was performed on a G-25 gel column.
   (4) An equal volume of 5 g/ml BSA protective solution was added to the solution of ABEI labeled with streptavitin to obtain the final solution.
**Preparation Example 11: Preparation of a solution of ABEI labeled with FITC**
   (1) Preparation of pH 9.5 dialysis solution: in a 5000ml beaker, 14.31g of Na₂CO₃ and 26.46g of NaHCO₃ was added, and filled up to 4500ml with water. The dialysate formulated was placed on a magnetic stirrer for later use.
   (2) A dialysis bag with an interception capacity of 14000 was chosen, a portion of which with appropriate size was prepared for later use. 1mg of FITC was dissolved and adjusted to 1ml with the dialysis solution formulated above, and placed into the dialysis bag. Dialysis was performed under stirring for 2 hours, and 50 µg of ABEI-hemisuccinimide-N-Hydroxysuccinimide was added to the dialyzed solution for reaction at 37 °C for 2 hours to produce the solution of ABEI labeled with FITC.
   (3) Purification of the solution of ABEI labeled with FITC obtained in the above reaction was performed on a G-25 gel column.
   (4) An equal volume of 5 g/ml BSA protective solution was added to the purified solution of ABEI labeled with FITC to obtain the final solution.
**Preparation Example 12: Preparation of a low-point calibrator and a high-point calibrator of gastrin-17**
   Using the gastrin-17 standard and using bovine serium as a solvent, a high-point calibrator and a low-point calibrator of gastrin-17 were prepared at the concentrations of 229.932 pmol/l and 4.729 pmol/l, respectively.

### Example 1

Preparation of kit components:
preparing an solution of the first anti-gastrin-17 antibody labeled with ABEI according to the Preparation Example 4 above;
preparing a suspension of magnetic spheres coated with a second anti-gastrin-17 antibody according to the Preparation Example 1 above; and
preparing a low-point calibrator and a high-point calibrator of gastrin-17 according to the Preparation Example 12 described above.

The gastrin-17 standards were used to prepare standard solutions of ten different concentrations, with bovine serum as the solvent.

The ten solutions were tested using the kit components prepared in the present embodiment, the detection procedure being as follows:
1) adding 40 µl of a solution to be tested, the high-point calibrator, and the low-point calibrator to different assay wells;
2) adding 100 µl of the solution of the first anti-gastrin-17 antibody labeled with ABEI to each well;
3) adding 20 µl of the suspension of magnetic spheres coated with the second anti-gastrin-17 antibody to each well;
4) incubating under 37°C for 30 minutes, and cleaning in a magnetic environment for 3 times for each well;
5) adding luminescent substrate to each well and detecting optical signal intensity; and
6) calculating the gastrin-17 concentration in the subject sample using the optical signal intensity detected in the subject sample with reference to the calibrated working curve with the calibrator.

The test results are shown in Table 1.

In addition, fasting serum samples from 5 patients diagnosed of duodenal ulcer from Nanshan Hospital (Shenzhen, China) were used as subject samples. These five samples underwent determination using the kit components provided in the present embodiment according to the detection method above. The test results are shown in Table 2.

### Example 2

Preparation of kit components:
preparing an solution of the first anti-gastrin-17 antibody labeled with ABEI according to the Preparation Example 4 above;
preparing a suspension of magnetic spheres coated with goat anti-FITC polyclonal antibody according to the Preparation Example 3 above;
preparing a solution of the second anti-gastrin-17 antibody labeled with FITC according to the Preparation Example 6 above; and
preparing a low-point calibrator and a high-point calibrator of gastrin-17 according to the Preparation Example 12 described above.

In addition, the ten standard solutions from Example 1 and the serum samples from 5 patients diagnosed of duodenal ulcer underwent determination for gastrin-17 using the kit components provided in the present embodiment by chemiluminesent Immunoassay with the detection method according to Example 1. The test results are shown in Table 1 and Table 2, respectively.

### Example 3

Preparation of kit components:
preparing a solution of the first anti-gastrin-17 antibody labeled with ABEI according to the Preparation Example 4 above;
preparing a biotinylated solution of the second anti-gastrin-17 antibody according to the Preparation Example 5 above;
preparing a suspension of magnetic spheres coated with streptavitin according to the Preparation Example 2 above; and
preparing a low-point calibrator and a high-point calibrator of gastrin-17 according to the Preparation Example 12 described above.

In addition, the ten standard solutions from Example 1 and the serum samples from 5 patients diagnosed of duodenal ulcer underwent determination for gastrin-17 using the kit components provided in the present embodiment by chemiluminesent Immunoassay with the detection method according to Example 1. The test results are shown in Table 1 and Table 2, respectively.

### Example 4

Preparation of kit components:
preparing a biotinylated solution of the first anti-gastrin-17 antibody according to the Preparation Example 5 above;
preparing a solution of streptavitin labeled with ABEI according to the Preparation Example 10 above;
preparing a suspension of magnetic spheres coated with the second anti-gastrin-17 antibody according to the Preparation Example 1 above; and
preparing a low-point calibrator and a high-point calibrator of gastrin-17 according to the Preparation Example 12 described above.

In addition, the ten standard solutions from Example 1 and the serum samples from 5 patients diagnosed of duodenal ulcer underwent determination for gastrin-17 using the kit components provided in the present embodiment by chemiluminesent Immunoassay with the detection method according to Example 1. The test results are shown in Table 1 and Table 2, respectively.

### Example 5

Preparation of kit components:
preparing a solution of the first anti-gastrin-17 antibody labeled with ABEI according to the Preparation Example 4 above;
preparing a suspension of magnetic spheres coated with the gastrin-17 antigen according to the Preparation Example 1 above; and
preparing a low-point calibrator and a high-point calibrator of gastrin-17 according to the Preparation Example 12 described above.

In addition, the ten standard solutions from Example 1 and the serum samples from 5 patients diagnosed of duodenal ulcer underwent determination for gastrin-17 using the kit components provided in the present embodiment by chemiluminesent Immunoassay with the detection method according to Example 1. The test results are shown in Table 1 and Table 2, respectively.

### Example 6

Preparation of kit components:
preparing a solution of the first anti-gastrin-17 antibody labeled with ABEI according to the Preparation Example 4 above;
preparing a suspension of magnetic spheres coated with goat anti-FITC polyclonal antibody according to the Preparation Example 3 above;
preparing a solution of gastrin-17 antigen labeled with FITC according to the Preparation Example 6 above; and
preparing a low-point calibrator and a high-point calibrator of gastrin-17 according to the Preparation Example 12 described above.

In addition, the ten standard solutions from Example 1 and the serum samples from 5 patients diagnosed of duodenal ulcer underwent determination for gastrin-17 using the kit components provided in the present embodiment by chemiluminesent Immunoassay with the detection method according to Example 1. The test results are shown in Table 1 and Table 2, respectively.

### Example 7

Preparation of kit components:
preparing an solution of the first anti-gastrin-17 antibody labeled with ABEI according to the Preparation Example 4 above
preparing a biotinylated solution of gastrin-17 antigen according to the Preparation Example 5 above;
preparing a suspension of magnetic spheres coated with streptavitin according to the Preparation Example 2 above; and
preparing a low-point calibrator and a high-point calibrator of gastrin-17 according to the Preparation Example 12 described above.

In addition, the ten standard solutions from Example 1 and the serum samples from 5 patients diagnosed of duodenal ulcer underwent determination for gastrin-17 using the kit components provided in the present embodiment by chemiluminesent Immunoassay with the detection method according to Example 1. The test results are shown in Table 1 and Table 2, respectively.

### Example 8

Preparation of kit components:
preparing a biotinylated solution of the first anti-gastrin-17 antibody according to the Preparation Example 5 above;
preparing a solution of streptavitin labeled with ABEI according to the Preparation Example 10 above;
preparing a suspension of magnetic spheres coated with gastrin-17 antigen according to the Preparation Example 1 above; and
preparing a low-point calibrator and a high-point calibrator of gastrin-17 according to the Preparation Example 12 described above.

In addition, the ten standard solutions from Example 1 and the serum samples from 5 patients diagnosed of duodenal ulcer underwent determination for gastrin-17 using the kit components provided in the present embodiment by chemiluminesent Immunoassay with the detection method according to Example 1. The test results are shown in Table 1 and Table 2, respectively.

### Example 9

Preparation of kit components:
preparing a solution of the second anti-gastrin-17 antibody labeled with streptavitin according to the Preparation Example 7 above;
preparing a solution of biotinylated ABEI according to the Preparation Example 9 above;
preparing a suspension of magnetic spheres coated with gastrin-17 antigen according to the Preparation Example 1 above; and
preparing a low-point calibrator and a high-point calibrator of gastrin-17 according to the Preparation Example 12 described above.

In addition, the ten standard solutions from Example 1 and the serum samples from 5 patients diagnosed of duodenal ulcer underwent determination for gastrin-17 using the kit components provided in the present embodiment by chemiluminesent Immunoassay with the detection method according to Example 1. The test results are shown in Table 1 and Table 2, respectively.

### Example 10

Preparation of kit components:
preparing a solution of the second anti-gastrin-17 antibody labeled with the goat anti-FITC polyclonal antibody according to the Preparation Example 8 above;
preparing a solution of FITC labeled with ABEI according to the Preparation Example 11 above;
preparing a suspension of magnetic spheres coated with the gastrin-17 antigen according to the Preparation Example 1 above; and
preparing a low-point calibrator and a high-point calibrator of gastrin-17 according to the Preparation Example 12 described above.

In addition, the ten standard solutions from Example 1 and the serum samples from 5 patients diagnosed of duodenal ulcer underwent determination for gastrin-17 using the kit components provided in the present embodiment by chemiluminesent Immunoassay with the detection method according to Example 1. The test results are shown in Table 1 and Table 2, respectively.

### Comparative Example 1

The ten standard solutions from Example 1 and the serum samples from 5 patients diagnosed of duodenal ulcer were tested using the Gastrin-17 Enzyme-linked Immunodetection Assay kit from Biohit (Finland). The results were compared with those of Example 1-10 and listed in Table 1 and Table 2.

**Table 1**

| Preparaion Concentratoin (pmol/l) | Example 1 (pmol/l) | Example 2 (pmol/l) | Example 3 (pmol/l) | Example 4 (pmol/l) | Example 5 (pmol/l) | Example 6 (pmol/l) | Example 7 (pmol/l) | Example 8 (pmol/l) | Example 9 (pmol/l) | Example 10 (pmol/l) | Comparative Example 1 (pmol/l) |
|---|---|---|---|---|---|---|---|---|---|---|---|
| 0 | 0.165 | 0.341 | 0.352 | 0.349 | 0.331 | 0.465 | 0.478 | 0.452 | 0.589 | 0.612 | 0.756 |
| 5 | 4.963 | 5.561 | 5.564 | 5.574 | 5.561 | 5.817 | 5.874 | 5.963 | 6.189 | 6.209 | 6.75 |
| 10 | 10.09 | 11.52 | 10.98 | 11.06 | 10.86 | 11.56 | 11.49 | 11.60 | 12.19 | 12.79 | 13.54 |
| 20 | 19.94 | 19.86 | 19.62 | 20.54 | 20.64 | 19.72 | 19.63 | 19.87 | 19.80 | 19.65 | 19.86 |
| 40 | 40.27 | 41.89 | 42.03 | 38.87 | 39.47 | 38.12 | 41.69 | 41.87 | 40.87 | 41.52 | 37.61 |
| 80 | 79.24 | 77.23 | 82.48 | 78.36 | 79.04 | 82.69 | 83.47 | 83.97 | 80.79 | 85.87 | 72.86 |
| 100 | 100.58 | 96.47 | 96.12 | 96.47 | 97.7 | 93.4 | 92.4 | 92.1 | 91.65 | 90.87 | 86.97 |
| 200 | 198.5 | 193.4 | 193.7 | 192.8 | 193.7 | 189.2 | 187.3 | 188.7 | 176.9 | 174.7 | 138.9 |
| 300 | 295.7 | 290.6 | 290.7 | 289.7 | 290.4 | 267.4 | 266.3 | 260.7 | 207.8 | 201.6 | 197.6 |
| 500 | 493.7 | 483.5 | 481.7 | 484.7 | 483.7 | 456.8 | 455.7 | 432.1 | 359.7 | 349.7 | 286.4 |

As can be seen from Table 1, the determination results of the gastrin-17 standard solutions using the kit provided in the present disclosure (Examples 1-10) were more accurate than the commercial ELISA kit. The results in the low-value range (5 pmol/L or less) and high-value range (300 pmol/l or more) were significantly better than the enzyme-linked immunosuppressive results. That is, the determination results using the kit of the present disclosure are closer to the theoretical values of the samples prepared using the gastrin-17 calibrators compared to the results of the enzyme-linked immunosorbent assay, especially in the low-value range and high-value range.

As can be seen from the detection results of Examples 1 to 4, the detection results in Example 1 are particularly good (mainly reflected in that the detection results in the low- and high-value ranges were closer to the theoretical values). Similarly, among Examples 5 to 10, the detection results of Example 5 were much better, indicating that, in the gastrin-17 detection kits of the present disclosure, the kits with antigen and antibody directly labeled with a trace marker or directly coated on a magnetic sphere had better detection accuracy and sensitivity compared to labeling or coating of magnetic spheres via a FITC system or a streptavidin system. By comparing Examples 8 and 9, it was found that the use of the first anti-gastrin-17 antibody had better performance than the use of the second anti-gastrin-17 antibody in detection by the competition method, indicating that, of the two antibodies, the first antibody had greater affinity for gastrin-17 antigen. Comparing Examples 4 and 8, it was found that the detection results of the double antibody sandwich method and the competition method showed differences when the indirect labeling method was used. In addition, the detection results of Example 5 was simliar to those of Examples 2 to 4 but slightly weaker than that of Example 1, indicating that the double antibody sandwich method as in Example 1 had better specificity and sensitivity than the use of the competition method as in Example 5. However, the competition method only requires one antibody while the double antibody sandwich method requires two antibodies. Therefore, the competition method is more efficient in terms of materials.

**Table 2***

| Sample ID | Example 1 | Example 2 | Example 3 | Example 4 | Example 5 | Example 6 | Example 7 | Example 8 | Example 9 | Example 10 | Comparative Example 1 |
|---|---|---|---|---|---|---|---|---|---|---|---|
| 1 | 34.62 | 32.14 | 32.01 | 32.64 | 33.34 | 31.69 | 31.41 | 31.87 | 31.53 | 31.77 | 28.64 |
| 2 | 12.97 | 12.14 | 11.97 | 12.03 | 12.04 | 10.87 | 10.93 | 10.78 | 10.81 | 10.72 | 8.891 |
| 3 | 45.87 | 43.14 | 43.57 | 43.17 | 43.89 | 42.17 | 42.67 | 42.07 | 42.45 | 42.01 | 37.56 |
| 4 | 27.61 | 25.74 | 25.41 | 24.69 | 25.03 | 24.58 | 23.96 | 24.09 | 24.21 | 23.89 | 15.86 |
| 5 | 65.97 | 63.14 | 64.01 | 62.41 | 63.78 | 60.12 | 59.33 | 60.14 | 59.88 | 59.77 | 54.98 |

| | | | | | | | | | | | |
|---|---|---|---|---|---|---|---|---|---|---|---|
| *: The unit for determined concentration: pmol/l; Normal value ranges for fasting examination: 2 to 10pmol/l. | | | | | | | | | | | |

As can be seen from Table 2, among the five patients suffering from massive gastrin-17 secretion due to duodenal ulcer, only 4 cases were detected as abnormal by the enzyme-linked immunosorbent detection kit. However, all 5 cases were detected to be abnormal using the kits of Examples 1 to 10 of the present disclosure. Therefore, the kit provided herein has a higher accuracy. It can be seen that the sample pre-dilution has a significant influence on the detection sensitivity of a kit, which can easily interfere with the clinical diagnosis and prolong the experimental time. Thus, the present disclosure provides a gastrin-17 kit and a method for detecting gastrin-17 which better meet the need for faster and more effective results in modern clinical trials.

While the disclosure has been described in detail, modifications within the spirit and scope of the disclosure will be apparent to those skilled in the art. In addition, it should be understood that various aspects of the disclosure, various parts of the various embodiments, and various features recited may be combined or interchangeable in full or in part. In each of the specific embodiments above, those embodiments which refer to another embodiment may be suitably combined with other embodiments, as will be understood by those skilled in the art. Furthermore, it will be understood by those skilled in the art that the foregoing description is only for the purpose of illustration by way of example and is not intended to limit the disclosure.

## Claims

1. A kit for detecting gastrin-17, **characterized in that** it comprises a component A and a component B, wherein the component A is a first anti-gastrin-17 antibody labeled with a trace marker or coated on a magnetic sphere, the component B is a second anti-gastrin-17 antibody labeled with a trace marker or coated on a magnetic sphere; wherein either one of the components A and B is labeled with a trace marker and the other one is coated on a magnetic sphere; wherein the first anti-gastrin-17 antibody and the second anti-gastrin-17 antibody have different binding sites for binding with gastrin-17; and wherein the trace marker is at least one selected from the group consisting of luminol and derivatives thereof, isoluminol and derivatives thereof, acridinium esters;
wherein the trace marker directly labels the first or second anti-gastrin-17 antibody, and wherein the first or second anti-gastrin-17 antibody is directly coated on the magnetic sphere.

2. The kit according to claim 1, **characterized in that** the trace marker is N-(4-aminobutyl) -N-ethylisoluminol.

3. The kit according to claim 1, **characterized in that** the magnetic sphere is a complex of Fe₂O₃ or Fe₃O₄ magnetic particles and an organic polymeric material and has a particle size of 0.1 to 5 µm; and, the magnetic sphere is optionally modified by surface modification to carry one or more active functional groups.

4. The kit according to claim 1, **characterized in that**, in the kit, the concentration of the first anti-gastrin-17 antibody is 10 to 200 µg/ml, the concentration of the second anti-gastrin-17 antibody is 10 to 200 µg/ml, the concentration of the trace marker is 0.1 to 1 mg/ml, and the concentration of the magnetic sphere is 0.1 to 5 mg/ml.

5. A method for preparing a kit for detecting gastrin-17, **characterized in that** it comprises: directly labeling either one of a first anti-gastrin-17 antibody and a second anti-gastrin-17 antibody with a trace marker and directly coating the other one on a magnetic sphere, to obtain a kit including the first anti-gastrin-17 antibody labeled with the trace marker or coated on the magnetic sphere and the second anti-gastrin-17 antibody coated on the magnetic sphere or labeled with the trace marker, wherein the first anti-gastrin-17 antibody and the second anti-gastrin-17 antibody have different binding sites for binding with gastrin-17.

6. The method according to claim 5, **characterized in that** the trace marker is N-(4-aminobutyl) -N-ethylisoluminol.

7. The method according to claim 5, **characterized in that** the magnetic sphere is a complex of Fe₂O₃ or Fe₃O₄ magnetic particles and an organic polymeric material and has a particle size of 0.1 to 5 µm.

8. The method according to claim 5, **characterized in that** in the prepared kit, the concentration of the first anti-gastrin-17 antibody is 10 to 200 µg/ml, the concentration of the second anti-gastrin-17 antibody is 10 to 200 µg/ml, the concentration of the trace marker is 0.1 to 1 mg/ml, and the concentration of the magnetic sphere is 0.1 to 5 mg/ml.

9. A method for detecting gastrin-17, **characterized in that** the method comprises using a gastrin-17 detection kit to detect gastrin-17 concentration in a subject sample by chemiluminescence immunoassay, the kit including a component A and a component B, wherein the component A is a first anti-gastrin-17 antibody labeled with a trace marker or coated on a magnetic sphere, the component B is a second anti-gastrin-17 antibody labeled with a trace marker or coated on a magnetic sphere; wherein either one of the components A and B is labeled with a trace marker and the other one is coated on a magnetic sphere; and, wherein the first anti-gastrin-17 antibody and the second anti-gastrin-17 antibody have different binding sites for binding with gastrin-17;
wherein the trace marker directly labels the first or second anti-gastrin-17 antibody, and wherein the first or second anti-gastrin-17 antibody is directly coated on the magnetic sphere.

10. The method of claim 9, **characterized in that** the method comprises: mixing the components A and B of the kit with the subject sample, incubating, magnetically separating, and adding a luminescent substrate to a resulting precipitate to detect an optical signal intensity; measuring the optical signal intensity of the low-point calibrator and the high-point calibrator of gastrin-17 in the same manner to obtain a standard curve between the gastrin-17 concentration and the optical signal intensity; and, comparing the optical signal intensity of the subject sample with the standard curve to obtain the gastrin-17 concentration in the subject sample.

11. The method according to claim 9 **characterized in that** the trace marker is N-(4-aminobutyl) -N-ethylisoluminol.

12. The method according to claim 9, **characterized in that** the magnetic sphere is a complex of Fe₂O₃ or Fe₃O₄ magnetic particles and an organic polymeric material and has a particle size of 0.1 to 5 µm.

13. The method according to claim 9, **characterized in that** in the prepared kit, the concentration of the first anti-gastrin-17 antibody is 10 to 200 µg/ml, the concentration of the second anti-gastrin-17 antibody is 10 to 200 µg/ml, the concentration of the trace marker is 0.1 to 1 mg/ml, and the concentration of the magnetic sphere is 0.1 to 5 mg/ml.

## Patentansprüche

1. Kit zur Detektion von Gastrin-17, **dadurch gekennzeichnet, dass** es eine Komponente A und eine Komponente B umfasst, worin die Komponente A ein erster Anti-Gastrin-17-Antikörper ist, der mit einem Spurenmarker markiert oder auf einer magnetischen Kugel beschichtet ist, worin die Komponente B ein zweiter Anti-Gastrin-17-Antikörper ist, der mit einem Spurenmarker markiert oder auf einer magnetischen Kugel beschichtet ist; worin die eine der Komponenten A und B mit einem Spurenmarker markiert ist und die andere Komponente auf einer magnetischen Kugel beschichtet ist; worin der erste Anti-Gastrin-17-Antikörper und der zweite Anti-Gastrin-17-Antikörper unterschiedliche Bindungsstellen zur Bindung mit Gastrin-17 haben; und worin der Spurenmarker zumindest einer ist, der aus der Gruppe ausgewählt wird, die aus Luminol und dessen Derivaten, Isoluminol und dessen Derivaten, Acridiniumestern besteht;
worin der Spurenmarker den ersten oder den zweiten Anti-Gastrin-17-Antikörper direkt markiert, und worin der erste oder der zweite Anti-Gastrin-17-Antikörper auf der magnetischen Kugel direkt beschichtet ist.

2. Kit nach Anspruch 1, **dadurch gekennzeichnet, dass** der Spurenmarker N-(4-Aminobutyl)-N-Ethylisoluminol ist.

3. Kit nach Anspruch 1, **dadurch gekennzeichnet, dass** die magnetische Kugel ein Komplex aus magnetischen Teilchen aus Fe₂O₃ oder Fe₃O₄ und einem organischen Polymermaterial ist und eine Teilchengröße von 0,1 bis 5 µm hat; und die magnetische Kugel optional durch Oberflächenmodifizierung geändert wird, um eine oder mehrere aktive funktionelle Gruppen zu tragen.

4. Kit nach Anspruch 1, **dadurch gekennzeichnet, dass** im Kit die Konzentration des ersten Anti-Gastrin-17-Antikörpers 10 bis 200 µg/ml beträgt, die Konzentration des zweiten Anti-Gastrin-17-Antikörpers 10 bis 200 µg/ml beträgt, die Konzentration des Spurenmarkers 0,1 bis 1 mg/ml beträgt, und die Konzentration der magnetischen Kugel 0,1 bis 5 mg/ml beträgt.

5. Verfahren zur Herstellung eines Kits zur Detektion von Gastrin-17, **dadurch gekennzeichnet, dass** es umfasst: direktes Markieren des einen ersten Anti-Gastrin-17-Antikörpers und des einen zweiten Anti-Gastrin-17-Antikörpers mit einem Spurenmarker und direktes Beschichten des anderen auf einer magnetischen Kugel, um ein Kit zu erhalten, das den ersten Anti-Gastrin-17-Antikörper, der mit dem Spurenmarker markiert oder auf der magnetischen Kugel beschichtet ist, und den zweiten Anti-Gastrin-17-Antikörper, der auf der magnetischen Kugel beschichtet oder mit dem Spurenmarker markiert ist, umfasst, worin der erste Anti-Gastrin-17-Antikörper und der zweite Anti-Gastrin-17-Antikörper unterschiedliche Bindungsstellen zur Bindung mit Gastrin-17 haben.

6. Verfahren nach Anspruch 5, **dadurch gekennzeichnet, dass** der Spurenmarker N-(4-Aminobutyl)-N-Ethylisoluminol ist.

7. Verfahren nach Anspruch 5, **dadurch gekennzeichnet, dass** die magnetische Kugel ein Komplex aus magnetischen Teilchen aus Fe₂O₃ oder Fe₃O₄ und einem organischen Polymermaterial ist und eine Teilchengröße von 0,1 bis 5 µm hat.

8. Verfahren nach Anspruch 5, **dadurch gekennzeichnet, dass** im hergestellten Kit die Konzentration des ersten Anti-Gastrin-17-Antikörpers 10 bis 200 µg/ml beträgt, die Konzentration des zweiten Anti-Gastrin-17-Antikörpers 10 bis 200 µg/ml beträgt, die Konzentration des Spurenmarkers 0,1 bis 1 mg/ml beträgt, und die Konzentration der magnetischen Kugel 0,1 bis 5 mg/ml beträgt.

9. Verfahren zur Detektion von Gastrin-17, **dadurch gekennzeichnet, dass** das Verfahren die Verwendung eines Kits zur Detektion von Gastrin-17 umfasst, um eine Konzentration von Gastrin-17 in einer Subjektprobe durch Chemilumineszenz-Immunoassay zu detektieren, worin das Kit eine Komponente A und eine Komponente B umfasst, worin die Komponente A ein erster Anti-Gastrin-17-Antikörper ist, der mit einem Spurenmarker markiert oder auf einer magnetischen Kugel beschichtet ist, worin die Komponente B ein zweiter Anti-Gastrin-17-Antikörper ist, der mit einem Spurenmarker markiert oder auf einer magnetischen Kugel beschichtet ist; worin die eine der Komponenten A und B mit einem Spurenmarker markiert ist und die andere Komponente auf einer magnetischen Kugel beschichtet ist; worin der erste Anti-Gastrin-17-Antikörper und der zweite Anti-Gastrin-17-Antikörper unterschiedliche Bindungsstellen zur Bindung mit Gastrin-17 haben;
worin der Spurenmarker den ersten oder den zweiten Anti-Gastrin-17-Antikörper direkt markiert, und worin der erste oder der zweite Anti-Gastrin-17-Antikörper auf der magnetischen Kugel direkt beschichtet ist.

10. Verfahren nach Anspruch 9, **dadurch gekennzeichnet, dass** das Verfahren umfasst: Mischen der Komponenten A und B des Kits mit der Subjektprobe, Inkubieren, magnetisches Separieren und Hinzufügen eines lumineszierenden Substrats zu einem resultierenden Niederschlag, um eine optische Signalintensität zu detektieren; Messen der optischen Signalintensität des Tiefpunkt-Kalibrators und des Hochpunkt-Kalibrators von Gastrin-17 auf die gleiche Art und Weise, um eine Standard-Kurve zwischen der Konzentration von Gastrin-17 und der optischen Signalintensität zu erhalten; und Vergleichen der optischen Signalintensität der Subjektprobe mit der Standard-Kurve, um die Konzentration von Gastrin-17 in der Subjektprobe zu erhalten.

11. Verfahren nach Anspruch 9, **dadurch gekennzeichnet, dass** der Spurenmarker N-(4-Aminobutyl)-N-Ethylisoluminol ist.

12. Verfahren nach Anspruch 9, **dadurch gekennzeichnet, dass** die magnetische Kugel ein Komplex aus magnetischen Teilchen aus Fe₂O₃ oder Fe₃O₄ und einem organischen Polymermaterial ist und eine Teilchengröße von 0,1 bis 5 µm hat.

13. Verfahren nach Anspruch 9, **dadurch gekennzeichnet, dass** im hergestellten Kit die Konzentration des ersten Anti-Gastrin-17-Antikörpers 10 bis 200 µg/ml beträgt, die Konzentration des zweiten Anti-Gastrin-17-Antikörpers 10 bis 200 µg/ml beträgt, die Konzentration des Spurenmarkers 0,1 bis 1 mg/ml beträgt, und die Konzentration der magnetischen Kugel 0,1 bis 5 mg/ml beträgt.

## Revendications

1. Kit de détection de gastrine-17, **caractérisé en ce qu'**il comprend un composant A et un composant B, dans lequel le composant A est un premier anticorps anti-gastrine-17 marqué avec un marqueur de trace ou couché sur une sphère magnétique, le composant B est un second anticorps anti-gastrine-17 marqué avec un marqueur de trace ou couché sur une sphère magnétique ; dans lequel l'un des composants A et B est marqué avec un marqueur de trace et l'autre est couché sur une sphère magnétique ; dans lequel le premier anticorps anti-gastrine-17 et le second anticorps anti-gastrine-17 ont des sites de liaison différents pour liaison à la gastrine-17 ; et dans lequel le marqueur de trace est au moins un sélectionné dans le groupe comprenant le luminol et des dérivés de celui-ci, l'isoluminol et des dérivés de celui-ci, des esters d'acridinium ;
dans lequel le marqueur de trace marque directement le premier ou le second anticorps anti-gastrine-17, et dans lequel le premier ou le second anticorps anti-gastrine-17 est couché directement sur la sphère magnétique.

2. Kit selon la revendication 1, **caractérisé en ce que** le marqueur de trace est le N-(4-aminobutyl)-N-éthylisoluminol.

3. Kit selon la revendication 1, **caractérisé en ce que** la sphère magnétique est un complexe de particules magnétiques de Fe₂O₃ ou Fe₃O₄ et d'un matériau polymère organique et a une taille de particule de 0,1 à 5 µm ; et, la sphère magnétique est facultativement modifiée par modification de surface pour porter un ou plusieurs groupes fonctionnels actifs.

4. Kit selon la revendication 1, **caractérisé en ce que** dans le kit, la concentration du premier anticorps anti-gastrine-17 est de 10 à 200 µg/ml, la concentration du second anticorps anti-gastrine-17 est de 10 à 200 µg/ml, la concentration du marqueur de trace est de 0,1 à 1 mg/ml, et la concentration de la sphère magnétique est de 0,1 à 5 mg/ml.

5. Méthode de préparation d'un kit de détection de gastrine-17, **caractérisée en ce qu'**elle comprend les étapes consistant à : marqueur directement l'un d'un premier anticorps anti-gastrine-17 et d'un second anticorps anti-gastrine-17 avec un marqueur de trace et coucher l'autre directement sur une sphère magnétique, pour obtenir un kit comprenant le premier anticorps anti-gastrine-17 marqué au marqueur trace ou couché sur la sphère magnétique et le second anticorps anti-gastrine-17 couché sur la sphère magnétique ou marqué au marqueur trace, dans laquelle le premier anticorps anti-gastrine-17 et le second anticorps anti-gastrine-17 ont des sites de liaison différents pour liaison à la gastrine-17.

6. Méthode selon la revendication 5, **caractérisée en ce que** le marqueur de trace est le N(4-aminobutyl)-N-éthylisoluminol.

7. Méthode selon la revendication 5, **caractérisée en ce que** la sphère magnétique est un complexe de particules magnétiques de Fe₂O₃ ou Fe₃O₄ et d'un matériau polymère organique et a une taille de particule de 0,1 à 5 µm.

8. Méthode selon la revendication 5, **caractérisée en ce que** dans le kit préparé, la concentration du premier anticorps anti-gastrine-17 est de 10 à 200 µg/ml, la concentration du second anticorps anti-gastrine-17 est de 10 à 200 µg/ml, la concentration du marqueur de trace est de 0,1 à 1 mg/ml, et la concentration de la sphère magnétique est de 0,1 à 5 mg/ml.

9. Méthode de détection de gastrine-17, **caractérisée en ce que** la méthode comprend l'utilisation d'un kit de détection de gastrine-17 pour détecter une concentration de gastrine-17 dans un échantillon sujet par immunodosage par chimiluminescence, le kit comprenant un composant A et un composant B, dans lequel le composant A est un premier anticorps anti-gastrine-17 marqué avec un marqueur de trace ou couché sur une sphère magnétique, le composant B est un second anticorps anti-gastrine-17 marqué avec un marqueur de trace ou couché sur une sphère magnétique ; dans lequel l'un des composants A et B est marqué avec un marqueur de trace et l'autre est couché sur une sphère magnétique ; et, dans lequel le premier anticorps anti-gastrine-17 et le second anticorps anti-gastrine-17 ont des sites de liaison différents pour liaison à la gastrine-17 ;
dans lequel le marqueur de trace marque directement le premier ou le second anticorps anti-gastrine-17, et dans lequel le premier ou le second anticorps anti-gastrine-17 est couché directement sur la sphère magnétique.

10. Méthode selon la revendication 9, **caractérisée en ce que** la méthode comprend les étapes consistant à : mélanger les composants A et B du kit avec l'échantillon sujet, incuber, séparer magnétiquement et ajouter un substrat luminescent à un précipité résultant pour détecter une intensité de signal optique ; mesurer l'intensité du signal optique du calibrateur de point bas et du calibrateur de point haut de la gastrine-17 de la même manière pour obtenir une courbe standard entre la concentration de gastrine-17 et l'intensité de signal optique ; et, comparer l'intensité de signal optique de l'échantillon sujet avec la courbe standard pour obtenir la concentration de gastrine-17 dans l'échantillon sujet.

11. Méthode selon la revendication 9, **caractérisée en ce que** le marqueur de trace est le N-(4-aminobutyl)-N-éthylisoluminol.

12. Méthode selon la revendication 9, **caractérisée en ce que** la sphère magnétique est un complexe de particules magnétiques de Fe₂O₃ ou Fe₃O₄ et un matériau polymère organique et a une taille de particule de 0,1 à 5 µm.

13. Méthode selon la revendication 9, **caractérisée en ce que** dans le kit préparé, la concentration du premier anticorps anti-gastrine-17 est de 10 à 200 µg/ml, la concentration du second anticorps anti-gastrine-17 est de 10 à 200 µg/ml, la concentration du marqueur de trace est de 0,1 à 1 mg/ml, et la concentration de la sphère magnétique est de 0,1 à 5 mg/ml.
